# EUROPEAN PATENT APPLICATION

(11) **EP 1 688 470 A1**
(43) Date of publication of application: **09.08.2006**
(21) Application number: 05425053.5
(22) Date of filing: 04.02.2005
(51) Int. Cl.: C09D 201/00, C09D 139/06, C09D 175/04, C08G 83/00, C08L 39/06, C08L 101/00, C08L 75/04, C08J 7/04, A61L 29/08

(54) **Hydrophilic coated products and process for their production**

(71) Applicant: Bioservice S.p.A., 46025 Poggio Rusco (MN) (IT)
(72) Inventor: Berti, Corrado, 48022 Lugo (Ravenna) (IT); Lorenzetti, Cesare, 63013 Grottammare (Ascoli Piceno) (IT)
(74) Representative: Trupiano, Federica

(57) **Abstract**

A process of preparing hydrophilic coatings for plastic substrates comprises the steps of forming a primer on the substrate by treating it with a solution of TPU, preparing a mixture containing one or more hydrophilic polymers selected from polymers and copolymers of N-vinyl lactams and hydrophilic polyurethanes with one or more dendritic polymers and crosslinking said hydrophilic polymer on the layer of primer by means of said dendritic polymer(s).

## Description

The present invention relates to products provided with hydrophilic coating on plastic substrates, in particular polyvinyl chloride (PVC) substrates, and a process for the preparation of said coating. The invention also relates to the coating thus obtained.

More specifically, the invention relates to the preparation of a coating on substrates comprising biomedical devices to impart them low friction coefficient properties. The hydrophilic coating of the invention may be applied to a wide range of devices to obtain a low friction coefficient. Examples of devices that may be provided with coatings with a low friction coefficient are medical and biomedical devices such as catheters, endoscopes and laryngoscopes, probes for feeding, for drainage or for endotracheal use, tubes for extracorporeal blood circulation and different paramedical devices such as protections to place on lesions of various types, condoms, razors, etc.

Devices provided with a hydrophilic coating have been known for some time in the art.

The application of hydrophilic coatings to devices for biomedical use has in fact received considerable attention in order to increase biocompatibility of the device to which the coating is applied. A particularly important property of these coatings is the low friction coefficient that can be obtained with their application. This makes it possible to minimize the discomfort related to the insertion of devices such as catheters and probes, and the lesions that may occur during insertion or removal of the probe.

As described in the patent literature the methods of producing hydrophilic coatings can be initially divided into 5 different methods:
- simple coating with a hydrophilic polymer
- mixing or preparation of hydrophilic polymer compounds
- formation of interpenetrating polymer networks
- coating with hydrophilic polymers that can react chemically
- hydrophilic polymers "grafted" to the surface to be coated

The first four methods of coating have various disadvantages that limit the duration of the coating or make its preparation particularly complex.

The present invention relates to a process to chemically bind the polymer to the substrate to which the coating is applied, the inventors being of the opinion that this is the method that guarantees the best results in terms of quality of the coating (friction coefficient) and its duration.

Various methods have been used to chemically bind a hydrophilic polymer to a substrate.

In WO 89/09246 peroxides and activated photoinitiators are used to produce radicals by heating and by UV radiation respectively.

WO 98/58990 proposes a PVP coating in two layers with different degrees of cross-linking obtained by a polymeric photoinitiator and UV radiation.

GB 1600963 proposes the use of a solution of PVP and diisocyanate in various solvents (ethyl lactate, methyl ethyl ketone, caprolactone), to be applied to a polyurethane or PVP tube previously immersed in methylene chloride to obtain a certain degree of swelling. It is not clear if there are direct bonds with the PVP or merely the formation of an interpenetrating polymer network.

In patent US 4,373,009 a diisocyanate layer is first prepared, which is then covered with a hydrophilic layer in polyvinylpyrrolidone with polyacrylamide and cured at high temperatures.

In patent US 4,585,666 the technology described is proposed as a development of the 1981 patent GB 1 600 963. In particular, the coating is produced using PVP and oligomer isocyanates of the DESMODUR® class by BAYER, consisting of hexamethyl diisocyanate derivates.

Patent US 5,005,287 relates to a technique for preparing a hydrophilic layer with a lubricating effect on the surface of a razor. The hydrophilic layer is prepared by mixing N-vinyl pyrrolidone, polyvinylpyrrolidone and various other hydrosoluble polymers in various dosages in the presence of a radical photoinitiator.

The processes above described have some drawbacks; the high temperature required for cross-linking some of them may damage the plastic substrate, the cross-linking times are relatively long and the grip of the coating on the substrate is not always constant through time.

EP-A-1505102 in the name of the present applicant, which was filed on 07.08.2003 and which therefore concerns the present application only for novelty according to art. 54(3) EPC, describes a coating process that solves the problems illustrated above. According to said application, the coating of plastic substrates is obtained with a hydrophilic layer which provides for the use of a mixture of dendritic polymers with at least one hydrophilic polymer chosen from polyvinylpyrrolidone, polymers and copolymers of N-vinyl lactams and hydrophilic polyurethanes.

Said mixture can be used directly on the substrate if this is made of polyurethane and if therefore it has some affinity with the polymers present in the mixture; for substrates made of polyvinyl chloride, polyethylene and analogous hostile materials, i.e. with low affinity with the polymers of the mixture, the substrate must be previously treated to enable adhesion of the coating.

The aim of the present invention is to improve the coating technique for substrates in PVC or other hostile materials as specified in the application EP-A-1505102 and provide a process for producing a low friction coefficient hydrophilic polymer coating of a PVC, polyethylene or other hostile polymer substrate, which is fast, reliable and inexpensive and which provides a coating with high and constant properties of low friction coefficient.

This aim is achieved by means of the present invention, which relates to a process for the preparation of a hydrophilic coating for a substrate in plastic material, comprising the steps of mixing one or more hydrophilic polymers selected from polymers and copolymers of N-vinyl lactams and hydrophilic polyurethanes with one or more dendritic polymers and of cross-linking said hydrophilic polymer to said substrate by means of said dendritic polymer(s), characterised in that it further comprises the following steps:
- preparing a solution in polar solvents of a thermoplastic polyurethane not hostile to the hydrophilic coating polymers
- treating said substrate with said polyurethane solution, and
- applying said mixture of hydrophilic polymers to said treated substrate.

According to another aspect of the invention, the thermoplastic polyurethane in solution is a polyester or polyether segmented polyurethane. Preferably the hydrophilic polymer is chosen from polyvinylpyrrolidone (PVP) and polyvinylbutyral (PVB) and the dendritic structure polymer is a polyester.

According to a second aspect of the invention, the substrate is produced with a thermoplastic polyurethane (TPU) surface layer or in a PVC/TPU mixture.

According to one aspect of the invention the dendritic polymer is reacted with maleic anhydride to obtain functional groups that improve grafting of the hydrophilic polymer to the substrate.

A further subject of the invention is the coating obtained according to the above-mentioned process, comprising a hydrophilic layer containing a hydrophilic coating polymer and a dendritic polymer for grafting the coating polymer to the device to be coated, characterised in that it furthermore comprises a primer layer comprising a thermoplastic polyurethane located between said substrate and said hydrophilic layer.

The invention furthermore relates to biomedical devices coated with the coating layer described above.

The invention has numerous advantages with respect to the processes according to the known art. The use of a thermoplastic polyurethane based primer in combination with a dendritic polymer as a grafting unit has shown a reduction in cross-linking times, improved adhesion to the substrate and a lower friction coefficient. Furthermore the process for preparation of the coating is easier than in the known art.

As above mentioned, the present invention relates to the preparation of a hydrophilic coating layer for substrates in plastic material, said coating layer containing polymers and copolymers of N-vinyl lactams, in particular PVP and/or hydrophilic polyurethanes, and a dendritic polymer, functionalized or non-functionalized, that can be partially or totally mixed with the PVP, in order to improve the quality of the coating in terms of lubricity, duration and ease of preparation.

The plastic material of the substrate is the plastic normally used in known biomedical devices or in the devices mentioned above; besides polyurethane, the hydrophilic layer according to the invention may in fact be conveniently applied to many polymeric substrates such as polyethylene, polypropylene, polyesters, silicones, polyamides, elastomers based on polyester and polyamide, polyvinyl chloride, natural rubber, elastomers based on polyolefins.

The hydrophilic polymers to be used for the coating according to the invention are selected from polyvinylpyrrolidone and polymers and copolymers of N-vinyl lactams, and hydrophilic polyurethanes; preferred hydrophilic polyurethanes are derivatives of aliphatic or aromatic diisocyanates and polyethylene oxide or polyethylene glycol such as the product Tecogel ® by Surmodics.

Dendritic polymers suitable for use as grafting units of the hydrophilic polymer to the substrate are all those that can be mixed at least partially with said hydrophilic polymer, where with the wording "mixed at least partially" it is meant that after mixing and recovery by physical separation of the unmixed portion of dendritic polymer, at least 20% by weight of the dendritic polymer is incorporated in the hydrophilic coating polymer.

Dendritic polymers suitable for the purpose are polyesters, polyamidoamines (PAMAM), polyamines, polyethers and those based on polyurethane. The wording "dendritic polymers" is considered to include all those having a dendritic structure, irrespective of their synthesis method, including hyperbranched polymers obtained by polycondensation of ABx monomers.

Preferred dendritic polymers are the polyester ones and in particular Boltorn, a saturated polyester by Perstorp Polyols, and its derivates; these polymers are normally used in the preparation of photocrosslinkable paints.

The amount of dendritic polymer to be mixed with the hydrophilic polymer is within the range of 2% to 80% by weight of the weight of the dry hydrophilic polymer, that is 2 to 80 parts of dendritic polymer for each part of hydrophilic polymer. Preferred ranges are 15% to 50% and more preferably 20% to 40%. For polyvinylpyrrolidone the preferred range is 20% to 40% in weight and more preferably around 25-30% in weight.

The dendritic polymer can be functionalized by reaction with maleic anhydride or acrylic acid, methacrylic acid, their anhydrides and chlorides, e.g. as disclosed in WO02/22700. The object of functionalization is to provide unsaturations on the terminal groups of the dendritic structure of the polymer. This type of structure is particularly reactive in the presence of UV radiation, making it possible to obtain together with the PVP, or another hydrophilic polymer, persistent hydrophilic coatings in considerably short times; in particular, it was found that to cross-link polyvinylpyrrolidone and dendritic polymer functionalized with maleic anhydride on the polyurethane substrate only approximately 30 minutes were required and this time could be further reduced by increasing the power of the lamps used. A suitable lamp power is around 20W.

Surprisingly, it was noted that the dendritic polymer not functionalized with maleic anhydride also makes it possible to considerably reduce the times for cross-linking and formation of the hydrophilic layer in respect of prior art, as can be seen from the results of examples 4 and 5.

The invention also provides for the presence of at least one photoinitiator.

Suitable photoinitiators are, for example, benzophenone, 1-hydroxycyclohexyl phenyl ketone, 4-(2-hydroxyethoxy)phenyl, 2-hydroxy-2-methylpropyl ketone and their mixtures; suitable commercial products are Esacure (Lamberti) and Irgacure (BASF). The amount of photoinitiator by weight with respect to the weight of the dry hydrophilic polymer is within the range from 0.3 to 8.0% by weight of the dry hydrophilic polymer, that is from 0.3 to 8.0 parts by weight for 100 parts of dry hydrophilic polymer.

In the absence of dendritic polymer and photoinitiator the time required for an acceptable degree of cross-linking and adhesion of the PVP to the substrate are excessive and impossible to apply on an industrial scale.

The process according to the invention also includes the possibility of incorporating in the hydrophilic layer one or more agents capable of acting against bacterial growth, such as particles of elemental silver, silver salts, antibiotics, methylene blue, chelating compounds such as aminocarboxylic acids (for example EDTA), PVP compounds containing iodine (Povidone iodine), chlorhexydine salts, triclosan, quaternary ammonium salts such as benzalkonium chloride and anti-inflammatory agents such as betaine.

This incorporation occurs preferably by simply adding the compounds, or their mixture, to the solutions of the polymers that will form the coating of the invention. The added amount is from 2.0% to 45% by weight of the weight of the dry hydrophilic polymer, that is 2 to 45 parts by weight for 100 parts of dry hydrophilic polymer.

One or more polymers, preferably selected from carboxymethylcellulose, hydroxyethylcellulose, polyethylenglycol, polyacrylic acid, polymethacrylic acid, polyvinyl alcohol, polyvinylbutyral, polyacrylamide, cellulose acetate propionate, can be added to the above described mixture.

In the case of substrates very little (or not at all) functionalized, such as polyolefin or polyvinyl chloride, they are previously treated with a primer to make the substrate more receptive to the layer of coating and to facilitate cross-linking between substrate and coating by means of the dendritic polymer.

According to the invention, the layer of primer is obtained by treating the substrate, i.e. the biomedical device, with a solution of a thermoplastic polyurethane in polar solvents, drying the substrate and subsequently treating the same with the hydrophilic/dendritic mixture.

Suitable thermoplastic polyurethanes are segmented thermoplastic polyurethanes, for example ESTANE ® Texin ® , Desmopan ®, Elastollan ®, Lupranate ®, Desmodur ®, Irostic TM.

Suitable polar solvents are THF and acetone, with a ratio acetone:THF of 2:1 to 4:1, and preferably approximately 3:1. Preferably, the thermoplastic polyurethane is dissolved in THF and the solution thus obtained is diluted with acetone in the ratios described above. Other solvents that can be used are chosen from methylene chloride, chloroform, ethyl acetate, dimethyl formamide, diglime, trieline, trichloroethane and their mixtures. The use of THF/acetone is preferred because it does not damage the PVC substrate upon brief immersions, has low toxicity, quick drying times and high PVC/TPU compenetration.

As mentioned, preferential polyurethanes are those in the ESTANE® class; the quantity of TPU in solution in THF according to the invention is within the range of 0.8 to 0.18 g/ml, preferably 0.1 to 0.15 and more preferably is about 0.13 g/ml. After dilution with acetone the range is within 0.018 to 0.055 g/ml, preferably 0.02 to 0.045 g/ml.

The duration of treatment of the substrate in the TPU solution in THF/acetone depends on the substrate to be treated, generally treatment by immersion is between 30 seconds and 10 minutes; for a plasticated PVC substrate, i.e. a substrate such as the typical biomedical device, the immersion time is approximately 2 minutes.

The invention will now be described in greater detail with reference to the following non-limiting examples.

### Materials and methods:

Thermoplastic polyurethane ESTANE 58277
THF 99.9% (b.p. 65/67°C) (Aldrich)
Acetone 99% (Riedel de Haen)
Polyvinylpyrrolidone PVP K90 (Basf)
Absolute ethanol (Fluka)
Photoinitiators Esacure KIP 150 and Esacure KIP (Lamberti)
Boltorn H30 and Boltorn H40 (Perstorp Polyols)
Sodium Methoxide (Aldrich); Maleic Anhydride (Aldrich)

### Example A

### Treatment of the hostile substrate.

A primer is prepared by dissolving 0.325 g of polyurethane ESTANE 58277 in 2.5 ml of THF and diluting with 7.5 ml of acetone. A PVC tube (for example a catheter) is immersed in this solution for approximately two minutes and then dried with hot air. The coating obtained adheres to the PVC as PVC and PU give homogeneous mixtures.

The hydrophilic layer is deposited by a solution according to examples from 1 to 5 given below.

### Example 1

### Preparation of dendritic polyester functionalized with maleic anhydride.

15.28 grams of maleic anhydride and 1 ml of a solution (30% by weight) of sodium methoxide in methanol were added to 25 grams of Boltorn H30 dissolved in 80 ml of acetone; the mixture was left under stirring at ambient temperature for 2 days. The solution was subsequently placed in a rotavapor in order to recover the polymer functionalized with acid groups and unsaturations deriving from maleic anhydride. The resulting product, a highly viscous liquid obtained in this way, was used in a mixture with PVP to produce hydrophilic coatings.

### Example 2

### Preparation of a probe having a hydrophilic layer with functionalized dendritic polymers, without photoinitiator.

One gram of PVP was dissolved in 30 ml of absolute ethanol; to 7 ml of this solution 0.046 g of Boltorn H30 functionalized according to example 1 (20% by weight in respect of the PVP) and 3 ml of acetone were added. The solution thus obtained is opalescent.

A PVC probe treated with an Estane 58277 (Goodrich) polyurethane based primer was immersed in the above solution for 2 minutes, then dried with hot air and exposed for 30 minutes to the light of a UV lamp (wavelength between 200 and 300 nm) with a power of 20 W. After being wet with water the probe had an extremely low friction coefficient and excellent adhesion of the hydrophilic layer to the tube.

### Example 3

### Preparation of a probe having a hydrophilic layer with functionalized dendritic polymer and photoinitiator.

One gram of PVP was dissolved in 30 ml of absolute ethanol; to 7 ml of this solution 0.046 g of Boltorn H30 functionalized according to example 1 (20% in mass in respect of the PVP), 0.007 g of ESACURE KIP 150 and 3 ml of acetone were added.

A PVC probe treated with a Desmopan polyurethane based primer as in example A was immersed in the above solution for 2 minutes, then dried with hot air and exposed for 15 minutes to the light of a UV lamp (wavelength between 200 and 300 nm) with a power of 20 W. After being wet with water the probe had an extremely low friction coefficient and excellent adhesion of the hydrophilic layer to the tube.

### Example 4

### Preparation of a probe having a hydrophilic layer with non-functionalized dendritic polymer, with photoinitiator.

One gram of PVP was dissolved in 30 ml of absolute ethanol; to 7 ml of this solution 0.060 g of Boltorn H30 (26% in mass in respect of the PVP), 0.007 g of photoinitiator ESACURE KIP 150 (3% in mass in respect of the PVP) and 3 ml of acetone were added.

A PVC probe treated with an Estane 58277 (Goodrich) polyurethane based primer was immersed in the above solution for 2 minutes, then dried with hot air and exposed for 15 minutes to the light of a UV lamp (wavelength between 200 and 300 nm) with a power of 20 W. After being wet with water the probe had an extremely low friction coefficient and excellent adhesion of the hydrophilic layer to the tube.

### Example 5

### Preparation of a probe having a hydrophilic layer with non-functionalized dendritic polymer, with photoinitiator.

One gram of PVP was dissolved in 30 ml of absolute ethanol; to 7 ml of this solution 0.060 g of Boltorn H40 (26% in mass in respect of the PVP), 0.007 g of photoinitiator ESACURE KIP 150 (3% in mass in respect of the PVP) and 3 ml of acetone were added.

A PVC probe treated with an Estane 58277 (Goodrich) polyurethane based primer was immersed in the above solution for 2 minutes, then dried with hot air and exposed for 15 minutes to the light of a UV lamp (wavelength between 200 and 300 nm) with a power of 20 W. After being wet with water the probe had an extremely low friction coefficient and excellent adhesion of the hydrophilic layer to the tube. There were no significant differences in respect of the probe prepared according to example 4, notwithstanding the fact that the dendritic polymer was not functionalized.

As above mentioned, according to one aspect of the invention the TPU/PVC primer layer is obtained using a TPU/PVC mixture as material for production of the substrate. In particular the substrate can be produced entirely with said mixture or can be coextruded or comoulded in order to use the mixture only for one surface layer.

Alternatively, the layer of primer can be obtained with an isocyanate based prepolymer normally used for paints and adhesives, for example Desmodur produced by Bayer.

In all cases, the layer of polyurethane improves the biocompatibility of the device also without the hydrophilic layer and constitutes a very effective physical barrier against migration of the PVC plasticizers.

## Claims

1. A process of preparing hydrophilic coatings for substrates in plastic material, comprising the steps of preparing a mixture containing one or more hydrophilic polymers selected from polyvinylpyrrolidone and polymers and copolymers of N-vinyl lactams and hydrophilic polyurethanes with one or more dendritic polymers and of cross-linking said hydrophilic polymer onto said substrate by means of said dendritic polymer(s), **characterised in that** it further comprises the following steps:
preparation of a solution of a thermoplastic polyurethane in polar solvents,
treatment of said substrate with said polyurethane solution to form a layer of primer, and
application of said mixture of hydrophilic polymers to said substrate treated with the primer to form a layer of hydrophilic coating.

2. A process as claimed in claim 1, wherein said cross-linking step of the hydrophilic layer is carried out under UV radiation.

3. A process according to one of the previous claims, also comprising the step of functionalizing said dendritic polymer with unsaturations on the terminal groups of its chain.

4. A process according to one of the previous claims, also comprising the step of adding antibacterial and/or anti-inflammatory agents to said mixture.

5. A process according to one of the previous claims, wherein are mixed from 2 to 80 parts by weight of said dendritic polymer for 100 parts of hydrophilic polymer.

6. A process according to one of the previous claims, wherein said dendritic polymer is selected from polyesters and polyurethanes.

7. A process as claimed in claim 6, wherein said hydrophilic polymer is selected from polyvinylpyrrolidone and hydrophilic polyurethanes.

8. A process according to one of the previous claims, **characterised in that** said thermoplastic polyurethane to be dissolved in polar solvents is chosen from poly(ester)urethanes or poly(ether)urethanes.

9. A process as claimed in claim 8, **characterised in that** the quantity of thermoplastic polyurethane in solution is within the range of 0.018 to 0.055 g/ml, preferably 0.02 to 0.045 g/ml.

10. A hydrophilic coating as obtainable according to one of the previous claims and containing a layer of polyurethane primer and a layer of coating containing a hydrophilic polymer and a dendritic polymer.

11. A medical or paramedical device comprising a hydrophilic coating as obtainable according to one of the previous claims and containing a hydrophilic polymer and a dendritic polymer.

12. A medical or paramedical device as claimed in claim 11, wherein said hydrophilic coating is applied to a substrate that comprises at least one layer of PVC/TPU mixture.
